# EUROPEAN PATENT APPLICATION

(11) **EP 1 726 961 A1**
(43) Date of publication of application: **29.11.2006**
(21) Application number: 05719775.8
(22) Date of filing: 02.03.2005
(51) Int. Cl.: G01N 33/569

(54) **AGENT AND METHOD FOR EXAMINING INFECTION WITH ADULT TAENIASIS SOLIUM AND TAENIARHYNCHUS SAGINATUS**

(30) Priority: 17.03.2004 JP 2004075805
(71) Applicant: Japan Science and Technology Agency, Kawaguchi-shi, Saitama 332-0012 (JP)
(72) Inventor: NAKAO, Minoru, Asahikawa-shi, Hokkaido 0788804 (JP); ITO, Akira, Asahikawa-shi, Hokkaido 0788373 (JP)
(74) Representative: Wilson Gunn
(86) International application number: PCT/JP2005/003460
(87) International publication number: WO 2005/090989

(57) **Abstract**

Present invention provides a diagnostic means for testing infection of adult in humans parasitized with adult *Taenia solium* and *Taenia saginata.* It was found that a hydrophobic ligand binding protein (HLBP) derived from an adult separated from tapeworms has an antigenicity. The present invention is a clinical diagnostic reagent for testing infection of adult *Taenia solium* and *Taenia saginata,* comprising, as a main ingredient, (a) a peptide containing at least residues 22-65 of residues 20-85 of any amino acid sequence of SEQ ID NOs: 1 to 4 or (b) a peptide containing an amino acid sequence of SEQ ID NO: 5. It is possible to examine the adult infection of *Taenia solium* and *Taenia saginata* by reacting the clinical diagnostic reagent with serum from a subject (human).

## Description

### Field of the Invention

The present invention relates to a clinical diagnostic reagent and an diagnostic method for testing infection of adult *Taenia solium* and *Taenia saginata.*

### Prior Art

Among various Taeniidae tapeworms parasitic to humans, sanitarily important tapeworms involve *Taenia solium* and *Taenia saginata,* whose final hosts are humans; and *Echinococcus granulosus* and *Echinococcus multilocularis,* whose final hosts are dogs and foxes.
*Taenia solium* is "a kishimen (note: Japanese flat nooodle) -like tapeworm growing up to several meters", whose larvae, referred to as Cysticercus cellulosae, are immobilized inside muscles of pigs and others, are incorporated into humans together with pig meats with insufficiently heated on cooking, and are parasitic to human alimentary tract. Cycticercus cellulosaes (referred to as cysticercis) grow in entire body of humans and pigs, which incorporated orally worm eggs passed out from adult worm-bearing humans. The infection of worms essentially depends on ingestion of "pig meats infected with cysticercis". The life cycle of the parasitic worm is complete among humans (cysticercis → *Taenia solium →* worm eggs), pigs (worm eggs → cysticercis) and humans (cysticercis → *Taenia solium →* worm eggs). By preventing humans from "eating raw pig meat infected with cysticercis, it is possible to cut the life cycle of this parasitic worm in long-range. However, the problem of cysticercosis is on the infection of worm eggs passed out from worm-bearing humans (parasite carriers) not only to pigs but also to other humans.
Although *Taenia saginata* has similar structure and life cycle to those of *Taenia solium,* there are few symptoms and no cysticercis appears in human tissues.
To prove the infection of *Taenia solium* or *Taenia saginata* in final host, a general method has been detection of proglottis and worm eggs in feces. Recently, not only morphological detection of excremental matter from final hosts but also detection by use of intra-fecal antigen or DNA has been tried to identify tapeworm infection.

However, a practical diagnostic method for testing tapeworm infection by detecting a serum antibody has not been developed (Reference 1).
On the other side, a hydrophobic ligand binding protein (HLBP) is a low molecular weight protein with molecular weight of about 10 kDa specific to tapeworms and is considered as an essential component for life-sustaining by binding with lipids and vitamins. However, the detailed function has not been elucidated (Reference 2).
Reference 1: Kensa to Gijutsu (Testing and engineering), 26 (4), 391-393 (1988)
Reference 2: FEBS Letters 487 (2000) 181-184.

### Problems to be solved by the Invention

The present invention aims to provide a diagnostic method for testing adult worm infection without using stool test, when adult *Taenia solium* or *Taenia saginata* are parasitized in humans.

### Means to solve the Problems

It is generally considered that host antibodies are not easily produced against adult tapeworms parasitic in an alimentary tract. However, the present inventors found that hydrophobic ligand binding protein (HLBP) derived from adult tapeworms separated from tapeworms had antigenicity and accomplished the present invention.

Namely, the present invention is a clinical diagnostic reagent for testing infection of adult *Taenia solium* and *Taenia saginata,* comprising, as a main ingredient, (a) a peptide containing at least residues 22-65 of residues 20-85 of any amino acid sequence of SEQ ID NOs: 1 to 4 or (b) a peptide containing an amino acid sequence of SEQ ID NO: 5.
Furthermore, the present invention is a diagnostic method for testing infection of adult *Taenia solium* and *Taenia saginata,* comprising reacting serum of a subject with the clinical diagnostic reagent of claim 1.

Moreover, the present invention is a diagnostic method for testing infection of adult *Taenia solium* and *Taenia saginata,* comprising the steps of (i) fixing (a) a peptide comprising at least residues 22-65 of residues 20-85 of any amino acid sequence of SEQ ID NOs: 1 to 4 or (b) a peptide containing an amino acid sequence of SEQ ID NO: 5 on a support, (ii) subjecting the peptide on the support to react with serum from a subject, (iii) subjecting the reactant to react with a labeled probe, and (iv) detecting the label.
Still furthermore, the present invention is a diagnostic kit for testing infection of adult *Taenia solium* and *Taenia saginata* in serum from a subject, comprising (a) a peptide containing at least residues 22-65 of residues 20-85 of any amino acid sequence of SEQ ID NOs: 1 to 4 or (b) a peptide containing an amino acid sequence of SEQ ID NO: 5, fixed on a support.

### Effects of the Invention

The clinical diagnostic reagent (or peptide antigen) of the present invention is very useful for human serological diagnosis. Application of the clinical diagnostic reagent of the present invention to antibody assay system enables to determine whether or not adult tapeworms are infected by detecting a specific antibody from a subject.
Taeniasis by *Taenia solium* or by *Taenia saginata* is a slight disease with parasitic adult worm in alimentary tract. However, an infected patient with adult worms contaminates life environment as a shedder of worms and eggs without awareness. In the case of *Taenias solium,* particularly, larvae could invade into central nerve system when scattered worms and eggs are incorporated into others orally (Cysticercosis cellulosae).
Taeniasis by *Taenia solium* or by *Taenia saginata* is not a rare disease in developing countries and *Taenia solium* is sanitarily important because it frequently induces cysticercosis. Since humans are sole final hosts for these parasites, it is possible to eradicate them if carriers of adult worms are efficiently detected and mass antiparasitic is possible.
On the other hand, an indigenization of *Taenia solium* and *Taenia saginata* has not been recognized in developed countries, but crisis of secondary infection has been worried out because of a lot of influx of travelers and laborers from epidemic places of developing countries. The clinical diagnostic reagent and the diagnostic method of the present invention will contribute to promote the efficiency of mass health examination for immigrants from epidemic places.

### Brief Description of the Drawings

Fig. 1 is a restriction enzyme map of pET35b vector (Novagen, USA).
Fig.2 is an immunoblot of Example 1. Lane 1: CBD+Tsag_AHLBP_c20, Lane 2: CBD+Tsag_AHLBP_c35, Lane 3: CBD+Tsol_AHLBP_exon, Lane 4: only fusion partner CBD. A: serum from a carrier of adult *Taenia saginata,* B: serum from a carrier of adult *Taenia solium.*

### Detailed Description of the Invention

Hydrophobic ligand binding protein (HLBP) is a protein initially identified in adult *Taenia diminuta* and isolated for the first time from *Hymenolepis diminuta* (Hdim, SEQ ID NO: 6), a rat tapeworm, and *Moniezia expansa* (Mexp, SEQ ID NO: 7), a sheep tapeworm (Reference 1, 2403292A). Both of these proteins are derived from adult tapeworms.
Hdim- and Mexp-derived HLBP (AHLBP) are different from AHLBP of adult *Taenia saginata* (referred to as Tsag) obtained by the present inventors (40~49% homology).

Homology comparisons among Tsag_AHLBP_c20 (SEQ ID NO: 1, 8), Tsag_AHLBP_c35 (SEQ ID NO: 4, 11) obtained by the present inventors from adult *Taenia saginata* and exon (Tsol_exon, SEQ ID NO: 5, 12) from adult *Taenia solium* are shown in Table 1.

**Table 1**

| % | Tsag_c35 | Tsol_exon |
|---|---|---|
| Tsag_AHLBP_c20 | 94 | 89 |
| Tsag_AHLBP_c35 | - | 82 |

The adult HLBP (AHLBP) of Tsag is very similar to that of Tsol (89~82% homology). The high degree of homology means that *Taenia saginata-*derived adult HLBP could be used as an antigen to detect both antibodies in Taeniasis of *Taenia saginata* and *Taenia solium.*

The present inventors compared the expression of HLBP gene profiles by RT-PCR in larval and adult *Taenia saginata* as a model. The results confirmed that various larval HLBP genes expressed in larvae are not expressed in adults. Then, screening cDNA library of adult *Taenia saginata,* the inventors succeeded in cloning an adult HLBP gene, which has different homology from larval HLBP genes (Examples 1 and 2).

It was beyond expectation of the inventors that HLBP molecules belonging to the same family present in larvae and adults and that their expression is strictly controlled in larvae and adults. The above fact means that each HLBP molecule used as antigen could distinctively detect antibody response of larvae from that of adults. As shown in later Examples, a recombinant protein of cloned adult HLBP gene was prepared in *E. coli* and was reacted with serum from an infected patient with *Taenia saginata* or *Taenia solium.* The result showed that adult HLBP molecule had antigenicity and could be applied to diagnostic purpose.

It is possible to examine whether or not *Taenia saginata* or *Taenia solium* is parasitic to a subject (human), by using the clinical diagnostic reagent (antigen) of the present invention and by reacting the clinical diagnostic reagent with the antibody produced in the subject parasitized with *Taenia saginata* or *Taenia solium.* Specimen materials involve serum, urine and preferably serum. If *Taenia saginata* or *Taenia solium* is parasitic to a subject, antibodies based on the parasites are produced in their serum and the antibodies react with the antigen. There are no restrictions in the method to detect the antigen-antibody reaction, however, the method involves immunoblot, dot blot, ELISA, others and preferably ELISA method.

Preferable diagnostic method foe esamining *Taenia saginata* or *Taenia solium* comprises the steps of fixing the antigen of the present invention on a solid support, subjecting the antigen on the support to react with serum from a subject, subjecting the reactant to react with a labeled probe, and detecting the label. It is preferable to insert appropriately a step of washing between each step.
Although there are no restrictions on the support, it is possible to use polystyrene and others as the support.
The antigen is subjected to react with antibodies present in the serum from a subject (with parasitic *Taenia saginata* or *Taenia solium*), and then the reactant is subjected to react with a probe recognizing the antibodies. The probe involves antihuman-IgG-antibody, protein G, protein A, protein L and others. Usually, these probes are labeled. The label involves a radioisotope (¹²⁵I) and enzymes (peroxidase, alkaline phosphatase). An enzyme antibody involves observation of color change (staining) after the reaction with a substrate.
The antigen of the present invention is possible to be synthesized, is stably and simply provided as a diagnostic antigen, and is easily subject to quality management.

The following Examples illustrate the present invention but are not intend to limit the scope of the present invention.

### Preparation Example 1

Larval cDNA and adult cDNA were prepared by reverse transcription, using oligo dT primer (SEQ ID NO: 13) with a tag sequence, from mRNA, each of which was prepared from larval *Taenia saginata* grown in an immunodeficient mice or adult *Taenia saginata* dewormed from a Chinese. PCR was performed using larval cDNA as a template, a forward primer (SEQ ID NO: 14) prepared from a signal sequence of a larval HLBP and a reverse primer for the tag sequence (SEQ ID NO: 15) and the PCR products were cloned. It was found from the result that larval HLBP had enormous polymorphism. However, no PCR product was obtained when PCR was performed using adult cDNA as a template and a forward primer (SEQ ID NO: 14) derived from larval HLBP.
A degenerate primer (TTYTAYGAYGARGAYCCNYT, SEQ ID NO: 17) was prepared from a FYDEDPL (SEQ ID NO: 16) part, which has a conserved amino acid sequence in adult HLBP (Reference 1, 2403292A) of *Taenia diminuta.* PCR was performed using the above adult *Taenia saginata* cDNA as a template, the degenerate primer and the reverse primer for the tag sequence (SEQ ID NO: 15).

It was found from the base sequence of the PCR product that HLBP was amplified, and then a reverse primer (SEQ ID NO: 18) was prepared from the region of stop codon of the sequence. PCR amplification was performed using lytic phage of adult *Taenia saginata* cDNA library, which was prepared by phage vector stored in the laboratory of the inventors, as a template. At the time of the amplification, the sequence proximal to a cloning site of the vector (SEQ ID NO: 19) was used as a forward primer and the newly prepared sequence proximal to HLBP (SEQ ID NO: 18) as a reverse primer. The amplified products were cloned and the clones containing full size were selected. Then, a forward primer (SEQ ID NO: 20) was prepared from a signal sequence. HLBP full length was PCR amplified using adult *Taenia saginata* cDNA as a template, using the forward primer and the reverse primer (SEQ ID NO: 15) derived from a tag sequence. Only the following four polymorphs were observed as the cloning result: Tsag_AHLBP_c20 (SEQ ID NO: 8, corresponding amino acid SEQ ID NO: 1), Tsag_AHLBP_c25 (SEQ ID NO: 9, corresponding amino acid SEQ ID NO: 2), Tsag_AHLBP_c27 (SEQ ID NO: 10, corresponding amino acid SEQ ID NO: 3), and Tsag_AHLBP_c35 (SEQ ID NO: 11, corresponding amino acid SEQ ID NO: 4) were obtained. The nucleotide sequence corresponding to residues 1-19 of these amino acid sequences was a putative signal sequence.

On the other hand, PCR was performed using genome DNA of *Taenia solium* collected from China as a template, the forward primer (SEQ ID NO: 20) derived from the signal sequence of Tsag_AHLBP and the reverse primer (SEQ ID NO: 18) obtained proximal to stop codon to amplify Tsol_AHLBP_exon (the bases 121-253 of SEQ ID NO: 12 (corresponding amino acid sequence SEQ ID NO: 5). The exon region was conjectured based on its high degree of homology to Tsag_AHLBP.

### Example 1

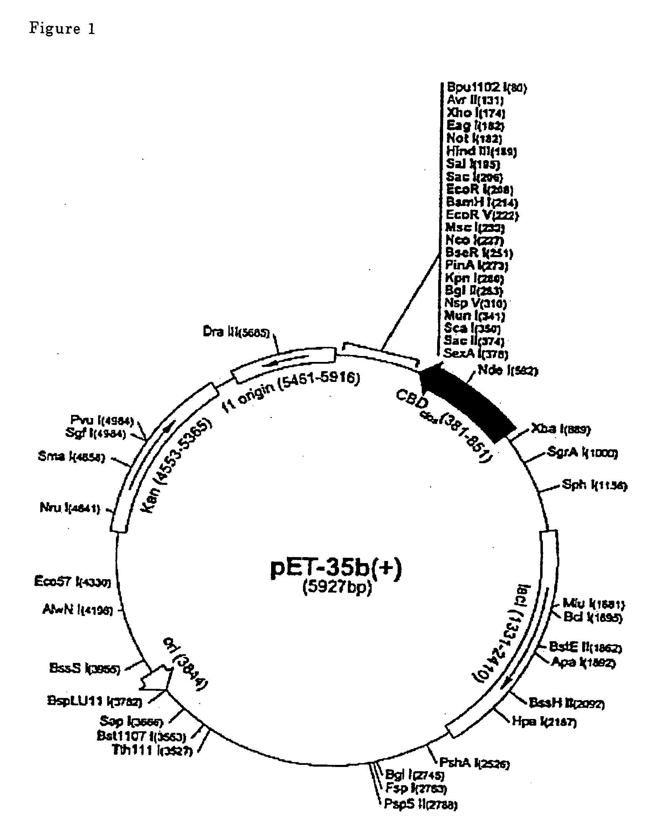

The sequences of Tsag_AHLBP_c20 (the bases 64-195 of SEQ ID NO: 8), Tsag_AHLBP_c35 (the bases 64-195 of SEQ ID NO: 11), Tsol_AHLBP_exon (the bases 121-253 of SEQ ID NO: 12) obtained in Preparation Example 1 were integrated into the EcoRI, SalI site of pET35b vector (Novagen, USA, Fig. 1). Each of the recombinant vectors was tranfected to *E. coli* BL21(DE3) pLysS (Novagen) and fusion proteins with cellulose binding domain (CBD) were prepared.

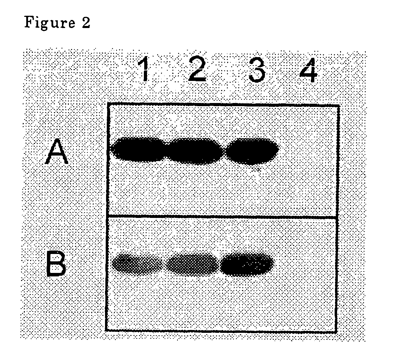

Figure 2 shows the photograph of the immunoblot obtained by the reaction between the fusion proteins and (A) serum from a carrier of adult *Taenia saginata* or (B) serum from a carrier of adult *Taenia solium.* Lanes 1 to 4 in Fig. 2 show (1) CBD+Tsag_AHLBP_c20, (2) CBD+Tsag_AHLBP_c35, (3) CBD+Tsol_AHLBP_exon, and (4) only fusion partner CBD, respectively. It was found that all serum reacted with AHLBP. It was also observed that carrier serum assessed as positive did not react with CBD, a fusion partner.

### Example 2

The recombinant proteins in Example 1 were reacted with serum of 3 infected patients with adult *Taenia saginata* and that of 2 infected patients with adult *Taenia solium* using the same method described in Example 1. It was assessed that 2 out of 3 infected patients with adult *Taenia saginata* (67%) and 2 out of 2 infected patients with adult *Taenia solium* (100%) were IgG antibody positive irrespective of fusion protein used. On the contrary, serum from one normal control was negative.

It was shown from these results that adult HLBP in *Taenia saginata* and *Taenia solium* had antigenicity. Furthermore, there are high degree of homology of amino acid sequence of adult HLBP between *Taenia solium* and *Taenia saginata.* Therefore, it was confirmed that adult HLBP of *Taenia saginata* could be used to detect the antibody of both infected patients with *Taenia solium* and those with *Taenia saginata.*

## Claims

1. A clinical diagnostic reagent for testing infection of adult *Taenia solium* and *Taenia saginata,* comprising, as a main ingredient, (a) a peptide containing at least residues 22-65 of residues 20-85 of any amino acid sequence of SEQ ID NOs: 1 to 4 or (b) a peptide containing an amino acid sequence of SEQ ID NO: 5.

2. A diagnostic method for testing infection of adult *Taenia solium* and *Taenia saginata,* comprising reacting serum of a subject with the clinical diagnostic reagent of claim 1.

3. A diagnostic method for testing infection of adult *Taenia solium* and *Taenia saginata,* comprising the steps of (i) fixing (a) a peptide comprising at least residues 22-65 of residues 20-85 of any amino acid sequence of SEQ ID NOs: 1 to 4 or (b) a peptide containing an amino acid sequence of SEQ ID NO: 5 on a support, (ii) subjecting the peptide on the support to react with serum from a subject, (iii) subjecting the reactant to react with a labeled probe, and (iv) detecting the label.

4. A diagnostic kit for testing infection of adult *Taenia solium* and *Taenia saginata* in serum from a subject, comprising (a) a peptide containing at least residues 22-65 of residues 20-85 of any amino acid sequence of SEQ ID NOs: 1 to 4 or (b) a peptide containing an amino acid sequence of SEQ ID NO: 5, fixed on a support.


SEQUENCE LISTING

<110> Japan Science and Technology Agency

<120> CLINICAL DIAGNOSTIC REAGENT AND DIAGNOSTIC METHOD FOR TESTING INFECTION OF ADULT TAENIA SOLIUM AND TAENIA SAGINATA

<130> FS05-425PCT

<160> 20

<170> PatentIn version 3.1

<210> 1
<211> 85
<212> PRT
<213> Taenia saginata

<400> 1

Met Gly Val Ala Ile Ile Ala Leu Leu Thr Leu Leu Ala Val Val Ala
1               5                   10                  15

Val Ser Ala Glu Glu Lys Asn Pro Val Arg Ala Ile Arg Ala Arg Cys
            20                  25                  30

Lys Glu Lys Val Ala Lys Trp Glu Lys Phe Phe Asp Glu Asp Pro Leu
        35                  40                  45

Gly Gln Lys Leu Ala Asp His Tyr Ala Lys Leu Arg Glu Leu Val Lys
    50                  55                  60

Glu Val Arg Leu Arg Ile Arg Lys Ala Ile Ala Lys Tyr Val Lys Glu
65                  70                  75                  80

Leu Lys Asp Glu Asn
                85

<210> 2
<211> 85
<212> PRT
<213> Taenia saginata

<400> 2

Met Gly Val Ala Ile Ile Ala Leu Leu Thr Leu Leu Ala Val Val Ala
1               5                   10                  15

Val Ser Ala Glu Glu Lys Asn Pro Val Arg Ala Ile Arg Ala Arg Cys
            20                  25                  30

Lys Glu Lys Val Ala Lys Trp Glu Lys Phe Phe Asp Glu Asp Pro Leu
        35                  40                  45

Gly Gln Lys Leu Ala Asp His Tyr Ala Lys Leu Arg Glu Leu Val Lys
    50                  55                  60

Glu Val Arg Leu Arg Ile Arg Lys Ala Ile Ala Lys His Val Lys Glu
65                  70                  75                  80

Leu Lys Asp Glu Asn
                85

<210> 3

<211>  85
<212>  PRT
<213>  Taenia saginata,

<400>  3

Met Gly Val Ala Ile Ile Ala Leu Leu Thr Leu Leu Ala Val Val Ala
1               5                   10                  15

Val Ser Ala Glu Glu Lys Asn Pro Val Arg Ala Ile Arg Ala Arg Cys
            20                  25                  30

Lys Glu Lys Val Ala Lys Trp Glu Arg Phe Phe Asp Glu Asp Pro Leu
        35                  40                  45

Gly Gln Lys Leu Ala Asp His Tyr Ala Lys Leu Arg Glu Leu Val Lys
    50                  55                  60

Glu Val Arg Leu Arg Ile Arg Lys Ala Ile Ala Lys Tyr Val Lys Glu
65                  70                  75                  80

Leu Lys Asp Glu Asn
                85


<210>  4
<211>  85
<212>  PRT
<213>  Taenia saginata

<400>  4

Met Gly Val Ala Ile Ile Ala Leu Leu Thr Leu Leu Ala Val Val Ala
1               5                   10                  15

Val Ser Ala Glu Glu Lys Asn Pro Val Arg Ala Ile Arg Ala Arg Cys
            20                  25                  30

Lys Glu Lys Val Ala Lys Trp Glu Lys Phe Phe Asp Glu Asp Pro Leu
        35                  40                  45

Gly Gln Lys Leu Ala Asp His Tyr Ala Asn Trp Ile Glu Leu Val Lys
    50                  55                  60

Glu Val Arg Leu Arg Ile Arg Lys Ala Ile Ala Lys His Val Lys Glu
65                  70                  75                  80

Leu Lys Asp Glu Asn
                85


<210>  5
<211>  44
<212>  PRT
<213>  Taenia solium

<400>  5

Lys Asn Pro Val Arg Val Ile Arg Thr Lys Tyr Lys Glu Lys Val Ala
1               5                   10                  15

Lys Trp Glu Lys Phe Phe Asp Glu Asp Pro Leu Gly Gln Lys Ile Ala
            20                  25                  30

Asp His Tyr Ala Lys Leu Arg Glu Leu Val Lys Glu
        35                  40

<210> 6
<211> 71
<212> PRT
<213> Hymenolepis diminuta

<400> 6

Met Glu Gln Glu Thr Asn Pro Ile Arg Arg Ile Lys Thr Lys Ala Lys
1               5                   10                  15

Glu Tyr Phe Ala Ala Arg Glu Arg Phe Tyr Asp Glu Asp Pro Leu Gly
            20                  25                  30

Lys Gln Ile Ala Ala His Leu Ser Lys Trp Arg Glu Ile Ile Arg Asp
        35                  40                  45

Val Arg Ala Arg Leu Arg Gly Tyr Leu Arg Lys Tyr Leu Asn Asp Leu
    50                  55                  60

Gln Lys Glu Tyr Pro Lys Ala
65                  70

<210> 7
<211> 67
<212> PRT
<213> Moniezia expansa

<400> 7

Met Glu Gln Glu Thr Asn Pro Ile Arg Ala Ile Lys Lys Arg Ile Thr
1               5                   10                  15

Ser Tyr Leu Lys Ser Arg Glu Glu Phe Tyr Asp Lys Asp Pro Leu Gly
            20                  25                  30

Gln Lys Ile Ala Lys Phe Tyr Gly Glu Trp Lys Glu Leu Val Ala Glu
        35                  40                  45

Val Arg Lys Arg Val Arg Ala Arg Ile Ala Ala Tyr Val Lys Lys Leu
    50                  55                  60

Gln Glu Glu
65

<210> 8
<211> 258
<212> DNA
<213> Taenia saginata

<400> 8
atgggagtcg ccatcatcgc tctcctcacc ctcctggctg tggtcgccgt gtctgctgaa 60

gagaagaacc cagttcgtgc tatccgagcc agatgcaagg agaaggtggc gaaatgggag 120

aagttctttg atgaggaccc tctaggccag aagcttgccg atcactatgc caagttgagg 180

gaactggtta aggaagtgag actacgtatt cgtaaggcca ttgcaaagta tgtgaaggag 240

ttaaaggatg agaactga 258

<210> 9

<211>  258
<212>  DNA
<213>  Taenia saginata

<400>  9
atgggagtcg ccatcatcgc tctcctcacc ctcctggctg tggtcgccgt gtctgctgaa      60

gagaagaacc cagttcgtgc tatccgagcc agatgcaagg agaaggtggc gaaatgggag     120

aagttctttg atgaggaccc tctaggccag aagcttgccg atcactatgc caagttgagg     180

gaactggtta aggaagtgag actacgtatt cgtaaggcca ttgcaaagca tgtgaaggag     240

ttaaaggatg agaactga                                                   258


<210>  10
<211>  258
<212>  DNA
<213>  Taenia saginata

<400>  10
atgggagtcg ccatcatcgc tctcctcacc ctcctggctg tggtcgccgt gtctgctgaa      60

gagaagaacc cagttcgtgc tatccgagcc agatgcaagg agaaggtggc gaaatgggag     120

aggttctttg atgaggaccc tctaggccag aagcttgccg atcactatgc caagttgagg     180

gaactggtta aggaagtgag actacgtatt cgtaaggcca ttgcaaagta tgtgaaggag     240

ttaaaggatg agaactga                                                   258


<210>  11
<211>  258
<212>  DNA
<213>  Taenia saginata

<400>  11
atgggagtcg ccatcatcgc tctcctcacc ctcctggctg tggtcgccgt gtctgctgaa      60

gagaagaacc cagttcgtgc tatccgagcc agatgcaagg agaaggtggc gaaatgggag     120

aagttctttg atgaggaccc tctaggccag aagcttgccg atcactatgc caattggata     180

gaactggtta aggaagtgag actacgtatt cgtaaggcca ttgcaaagca tgtgaaggag     240

ttaaaggatg agaactga                                                   258


<210>  12
<211>  310
<212>  DNA
<213>  Taenia solium

<400>  12
gtaagtgtac ttgtcattac cctaacccca ccttcgtctt ctagagaggt aagttcgtca      60

acttcgtcac attccacgtc catctttgca atatgcgaat ctaaacgtcg tccttgatag     120

aaaaacccag ttcgtgttat ccgaaccaag tacaaggaga aggtggcgaa atgggagaag     180

ttctttgatg aggatcctct gggccaaaag atagccgatc actatgccaa gttgagggaa     240

ctggttaagg aaggtaggtt tgtgttggac gaggctcctc cataacttgc atctccttct     300

ctcattttag                                                            310


<210>  13
<211>  40
<212>  DNA
<213>  Artificial sequence

<220>
<223>  tag

<400> 13
cacgtctaga ctgcagaagc tttttttttt tttttttttt 40

<210> 14
<211> 22
<212> DNA
<213> Artificial sequence

<220>
<223> pimer

<400> 14
atgcgtgcca cattttgctt ct 22

<210> 15
<211> 22
<212> DNA
<213> Artificial sequence

<220>
<223> primer

<400> 15
cacgtctaga ctgcagaagc tt 22

<210> 16
<211> 7
<212> PRT
<213> Taenia saginata

<400> 16

Phe Tyr Asp Glu Asp Pro Leu
1               5

<210> 17
<211> 20
<212> DNA
<213> Artificial sequence

<220>
<223> primer

<220>
<221> misc_feature
<222> (18)..(18)
<223> n means A, C, G or T.

<400> 17
ttytaygayg argayccnyt 20

<210> 18
<211> 23
<212> DNA
<213> Artificial sequence

<220>
<223> primer

<400> 18
tcagttctca tcctttaact cct 23

<210> 19
<211> 20
<212> DNA
<213> Artificial sequence

<220>

<223>  primer

<400>  19
cgaacgccag cacatggaca                                                    20


<210>  20
<211>  21
<212>  DNA
<213>  Artificial sequence

<220>
<223>  primer

<400>  20
atgggagtcg ccatcatcgc t                                                  21